(19) Europäisches Patentamt
European Patent Office
Office européen des brevets

(11) Veröffentlichungsnummer: **0 443 197 A2**

# (12) EUROPÄISCHE PATENTANMELDUNG

(21) Anmeldenummer: **90125408.6**

(22) Anmeldetag: **24.12.90**

(51) Int. Cl.5: **C07D 263/20**, A61K 31/42, C07D 413/06, C07D 413/14

(30) Priorität: **21.02.90 DE 4005371**

(43) Veröffentlichungstag der Anmeldung: **28.08.91 Patentblatt 91/35**

(84) Benannte Vertragsstaaten: **AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Prücher, Helmut**
**Königsbergerstrasse 9**
**W-6148 Heppenheim(DE)**
Erfinder: **Böttcher, Henning, Dr.**
**Soderstrasse 95**
**W-6100 Darmstadt(DE)**
Erfinder: **Gottschlich, Rudolf, Dr.**
**Buchenweg 1**
**W-6107 Reinheim(DE)**
Erfinder: **Minck, Klaus-Otto, Dr.**
**Büchestrasse 6**
**W-6105 Ober-Ramstadt(DE)**
Erfinder: **Seyfried, Chrsitoph, Dr.**
**Mathidenstrasse 6**
**W-6104 Seeheim(DE)**
Erfinder: **Haase, Anton, Dr.**
**Ahronweg 5**
**W-6109 Mühtal(DE)**

(54) **Oxazolidinone.**

(57) Neue Oxazolidinone der Formel I

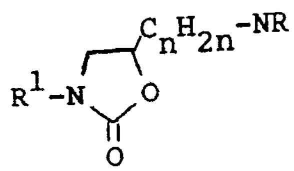

I

worin R, $R^1$ und n die in Patentanspruch 1 angegebene Bedeutung haben,
sowie deren Salze
zeigen das Zentralnervensystem beeinflussende, insbesondere dämpfende Wirkungen.

EP 0 443 197 A2

Die Erfindung betrifft neue Oxazolidinone der Formel I

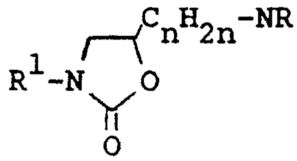

I

worin

R

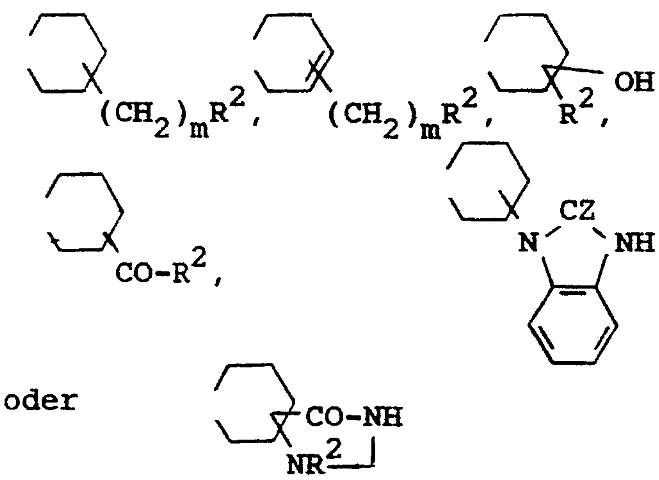

oder

| | |
|---|---|
| Z | O oder S, |
| $R^1$ und $R^2$ | jeweils unsubstituierte oder ein- oder zweifach durch Alkyl, Alkoxy, Alkylthio, Alkylsulfinyl oder Alkylsulfonyl mit jeweils 1-4 C-Atomen, Alkanoyloxy und/oder Alkanoylamino mit jeweils 1-6 C-Atomen, F, Cl, Br, OH und/oder $CF_3$ substituierte Phenyl- oder ein- oder zweikernige, 1-4 Heteroatome enthaltende Heteroarylreste, |
| m | 0 oder 1 und |
| n | 2 oder 3 |

bedeuten,
sowie deren Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dämpfende (z.B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologica 13 (1968), 222-257), hemmen bei Mäusen das durch Apomorphin induzierte Kletterverhalten (Methodik vgl. Costall et al., European J. Pharmacol. 50 (1968), 39-50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772-778, und Creese et al., European J. Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol. 33 (1975), 61-64). Weiterhin treten analgetische und blutdrucksenkende Wirkungen auf; so wird der bei kathetertragenden wachen, spontan hypertonen Ratten (Stamm SHR/NIH-MO//CHB-EMD; Methode vgl. Weeks und Jones, Proc.Soc.Exptl.Biol.Med. 104 (1960), 646-648) direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Oxazolidinone der Formel I sowie ihre Salze.

Gegenstand der Erfindung ist ferner ein Verfahren zur Herstellung von Oxazolidinonen der Formel I sowie von deren Salzen, dadurch gekennzeichnet, daß man eine Verbindung der Formel II

$Ox\text{-}C_nH_{2n}\text{-}X^1$ II

worin
Ox
den Rest

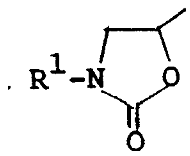 ,

| | |
|---|---|
| $X^1$ | X oder $NH_2$, |
| X | Cl, Br, J, OH oder eine reaktionsfähig funktionell abgewandelte OH-Gruppe bedeuten und |
| $R^1$ und n | die angegebenen Bedeutungen haben, mit einer Verbindung der Formel III |

$X^2\text{-}R\text{-}X^3$ III

worin

| | |
|---|---|
| $X^2$ und $X^3$ | gleich oder verschieden sind und, falls $X^1$ = $NH_2$ ist, jeweils X, andernfalls zusammen NH bedeuten und |
| R | die angegebene Bedeutung hat, |

umsetzt

und/oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

und/oder daß man zur Herstellung einer Verbindung der Formel I, worin

R 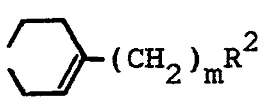 oder 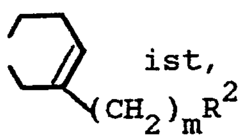 ist,

eine Verbindung der Formel IV

$Ox\text{-}C_nH_{2n}\text{-}R^5$

worin
$R^5$

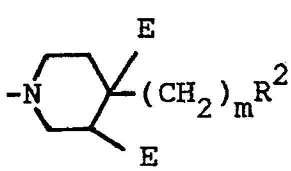

oder

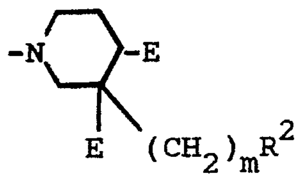 ,

der eine Rest E X, CN oder $NH_2$,
Der andere Rest E H bedeutet und
Ox, $R^2$, X und n die angegebenen Bedeutungen haben

mit einem HE-abspaltenden Mittel behandelt
oder daß man eine Verbindung der Formel V

$R^1$-NH-$CH_2$-CHOH-$C_nH_{2n}$-NR V

worin R, $R^1$ und n die angegebenen Bedeutungen haben, mit einem reaktionsfähigen Derivat der Kohlensäure umsetzt
und/oder daß man gegebenenfalls in einer Verbindung der Formel I eine O-Alkylgruppe unter Bildung einer OH-Gruppe spaltet und/oder eine Verbindung der Formel I durch Reduktion in eine andere Verbindung der Formel I umwandelt
und/oder daß man eine Base der Formel I durch Behandeln mit einer Säure in eines ihrer Salze umwandelt.

Verbindungen der Formel I umschließen die folgenden Verbindungen (worin x 2-, 3- oder 4-Stellung, y 2-, 3-, 4-, 5- oder 6-Stellung und z 6-, 7- oder 8-Stellung bedeutet):
die 3-$R^1$-5-(x-$R^2$($CH_2$)$_m$-piperidinoethyl- bzw. -propyl)-oxazolidin-2-one (Ia),
die 3-$R^1$-5-(y-$R^2$($CH_2$)$_m$-1,2,3,6-tetrahydropyridinoethyl-bzw. -propyl)-oxazolidin-2-one (Ib),
die 3-$R^1$-5-(x-$R^2$-x-hydroxy-piperidinoethyl- bzw. -propyl)-oxazolidin-2-one (Ic),
die 3-$R^1$-5-(x-$R^2$-CO-piperidinoethyl- bzw. -propyl)-oxazolidin-2-one (Id),
die 3-$R^1$-5-[x-(2-oxobenzimidazolin-1-yl)-piperidinoethyl- bzw. -propyl]-oxazolidin-2-one (Ie)
die 3-$R^1$-5-[x-(2-thioxobenzimidazolin-1-yl)-piperidinoethyl- bzw. -propyl]-oxazolidin-2-one (If)
die 1-$R^2$-4-oxo-z-(3-$R^1$-oxazolidin-2-on-5-yl-ethyl- bzw. -Propyl)-1,3,z-triazaspiro[4,5]-decane (Ig).

Bevorzugt sind Verbindungen der Formel Ic.

Formel Ia mit x = 2 (Iaa);
Formel Ia mit x = 3 (Iab);
Formel Ia mit x = 4 (Iac);

Formel Ib mit y = 2 (Iba);
Formel Ib mit y = 3 (Ibb);
Formel Ib mit y = 4 (Ibc);
Formel Ib mit y = 5 (Ibd);
Formel Ib mit y = 6 (Ibe);

Formel Ic mit x = 2 (Ica);
Formel Ic mit x = 3 (Icb);
Formel Ic mit x = 4 (Icc);

Formel Id mit x = 2 (Ida);
Formel Id mit x = 3 (Ibd);
Formel Id mit x = 4 (Idc);

Formel Ie mit x = 2 (Iea);
Formel Ie mit x = 3 (Ieb);
Formel Ie mit x = 4 (Iec);

Formel If mit x = 2 (Ifa);
Formel If mit x = 3 (Ifb);
Formel If mit x = 4 (Ifc);

Formel Ig mit z = 6 (Iga);
Formel Ig mit z = 7 (Igb);
Formel Ig mit z = 8 (Igc).

Bevorzugt sind Verbindungen der Formel Icc, ferner solche der Formeln Iaa, Iab, Iac, Ibc, Ica, Icb, Idc, Iec, Ife und Igc.

In den Resten $R^1$ bis $R^4$ bedeutet Alkyl vorzugsweise Methyl, ferner auch Ethyl, n-Propyl, Isopropyl, n-Butyl, Isobutyl, sek.-Butyl oder tert.-Butyl. Alkoxy ist vorzugsweise Methoxy, ferner auch Ethoxy, n-Propoxy, Isopropoxy, n-Butoxy, Isobutoxy, sek.-Butoxy oder tert.-Butoxy.

Die Reste $R^1$ und $R^2$ sind bevorzugt unsubstituiertes oder einfach substituiertes Phenyl. Falls $R^1$ bzw. $R^2$ substituierte Phenylgruppen bedeuten, so können sie jedoch auch zweifach substituiert sein, wobei die Substituenten gleich oder verschieden sein können. Bevorzugte Substituenten an den Phenylgruppen sind Methyl, Methoxy, F, Cl oder $CF_3$; ferner sind als Substituenten bevorzugt Ethyl, Ethoxy, Br und/oder OH. Im einzelnen sind $R^1$ und $R^2$ bevorzugt Phenyl, o-, m- oder p-Tolyl, o-, m- oder p-Methoxyphenyl, o-, m-oder p-Fluorphenyl, o-, m- oder p-Chlorphenyl, o-, m- oder p-Trifluormethylphenyl, ferner o-, m- oder p-Ethylphenyl, o-, m- oder p-Ethoxyphenyl, o-, m- oder p-Bromphenyl, o-, m- oder p-Hydroxyphenyl, 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dihydroxyphenyl, 2-Methyl-4-chlorphenyl.

Die Reste $R^1$ und $R^2$ können auch ein- oder zweikernige 1-4 Heteroatome enthaltende Heteroarylreste bedeuten, die vorzugsweise 5 oder 6 Ringglieder in jedem Ring enthalten. Als Heteroatome sind O, S und/oder N bevorzugt. Bevorzugte Heteroarylreste sind im einzelnen 2- oder 3-Furyl, 2- oder 3-Thienyl, 2-, 3- oder 4-Pyridyl, ferner 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 4-, 5- oder 6-Pyrimidinyl, weiterhin 1,2,3-Triazol-1-, -4- oder -5-yl, 1,2,4-Triazol-1-, -3-oder -5-yl, 1- oder 5-Tetrazolyl, 1,2,3-Oxadiazol-4-oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolinyl, 1-, 3-, 4-, 5-, 6-, 7- oder 8-Isochinolinyl, 3-, 4-, 5-, 6-, 7-oder 8-Chinolinyl, 2-, 4-, 5-, 6-, 7- oder 8-Chinazolinyl, 2-, 5- oder 6-Chinoxalinyl.

Der Rest $R^1$ ist ganz besonders bevorzugt p-Methoxyphenyl oder p-Fluorphenyl, der Rest $R^2$ Phenyl, p-Methoxyphenyl, p-Fluorphenyl, p-Chlorphenyl, m-Trifluormethylphenyl oder 2-Thienyl.

Z ist vorzugsweise O. Der Parameter m ist vorzugsweise O, so daß die Gruppe $R^2(CH_2)_m$- vorzugswiese die Bedeutung $R^2$ annimmt. Falls m = 1 ist, bedeutet die Gruppe $R^2CH_2$ vorzugsweise Benzyl, 2-, 3- oder insbesondere 4-Methoxybenzyl, 2-, 3- oder insbesondere 4-Chlorbenzyl.

Der Parameter n ist vorzugsweise 2. Die Gruppe $C_n H_{2n}$ steht bevorzugt für $(CH_2)_n$-, im einzelnen also bevorzugt für $-CH_2CH_2-$ oder $-CH_2CH_2CH_2-$, aber auch für $-CH(CH_3)-$, $-CH(CH_3)CH_2-$, $-CH_2CH(CH_3)-$ oder $-C(CH_3)_2$.

Dementsprechend sind Gegenstand der Erfindung insbesondere diejenigen Verbindungen der Formeln I bzw. Ia bis Igc, in denen mindestens einer der genannten Reste eine der vorstehend angegebenen, insbesondere der vorstehend angegebenen bevorzugten Bedeutungen hat. Einige bevorzugte Gruppen von Verbindungen entsprechen den vorstehend angegebenen Formeln, worin die nicht näher bezeichneten Reste und Parameter die bei den Formeln I bzw. Ia bis Igc angegebene Bedeutung haben, worin jedoch

(a) $R^1$ Phenyl, Tolyl, Methoxyphenyl, Fluorphenyl, Chlorphenyl, Hydroxyphenyl, Trifluormethylphenyl oder Dimethoxyphenyl bedeutet;

(b) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl oder 3,4-Dimethoxyphenyl bedeutet;

(c) $R^1$ p-Methoxyphenyl oder p-Fluorphenyl bedeutet;

(d) $R^2$ Phenyl, Tolyl, Methoxyphenyl, Fluorphenyl, Chlorphenyl, Trifluormethylphenyl oder Thienyl bedeutet;

(e) $R^2$ Phenyl, o-, m- oder p-Tolyl, p-Methoxyphenyl, p-Fluorphenyl, p-Chlorphenyl, m-Trifluormethylphenyl oder 2-Thienyl bedeutet;

(f) $R^2$ Phenyl, p-Methoxyphenyl, p-Chlorphenyl oder 2-Thienyl bedeutet;

(g) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl oder 3,4-Dimethoxyphenyl und
$R^2$ Phenyl, o-, m- oder p-Tolyl, p-Methoxyphenyl, p-Fluorphenyl, p-Chlorphenyl, m-Trifluormethylphenyl oder 2-Thienyl bedeuten;

(h) $R^1$ p-Methoxyphenyl und
$R^2$ Phenyl, p-Methoxyphenyl, p-Chlorphenyl oder 2-Thienyl bedeuten.

Im einzelnen sind bevorzugt alle Verbindungen der vorstehend genannten Formeln, in denen $R^1$ und/oder $R^2$ die vorstehend angegebenen bevorzugten Bedeutungen haben, worin ferner die Gruppe $-C_nH_{2n}$- $-CH_2CH_2$- und/oder gegebenenfalls der Parameter m 0 bedeutet.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Verbindungen der Formel I insbesondere durch Umsetzung von Verbindungen der Formeln Ox-$C_nH_{2n}$-Cl, Ox-$C_nH_{2n}$-Br oder Ox-$C_nH_{2n}$-$OSO_2CH_3$ mit Verbindungen der Formel III, worin $X^2$ und $X^3$ zusammen eine NH-Gruppe bedeuten (nachstehend als IIIa bezeichnet) erhältlich.

Die Verbindungen der Formel II und III sind zum Teil bekannt; die nicht bekannten Verbindungen der Formeln II und III können leicht analog zu den bekannten Verbindungen hergestellt werden. Primäre Alkohole der Formel Ox-$C_nH_{2n}$-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ox-$C_nH_{2n}$-Hal. Die entsprechenden Sulfonyloxyverbindungen sind erhältlich aus den Alkoholen Ox-$C_nH_{2n}$-OH durch Umsetzung mit den entsprechenden Sulfonsäurechloriden. Die Jodverbindungen der Formel Oc-$C_nH_{2n}$-J sind z.B. durch Einwirkung von Kaliumjodid auf die zugehörigen p-Toluolsulfonsäureester erhältlich. Die Amine der Formel Ox-$C_nH_{2n}$-$NH_2$ sind z.B. aus den Halogeniden mit Phthalimidkalium oder durch Reduktion der entsprechenden Nitrile herstellbar.

Die Verbindungen der Formel IIIa sind teilweise bekannt (vgl. DE-OS 20 60 816) und z.B. erhältlich durch Umsetzung von 2-, 3- oder 4-Piperidon mit metallorganischen Verbindungen der Formel M-$(CH_2)_mR^2$ (worin M ein Li-Atom oder MgHal bedeutet), anschließende Hydrolyse zu den entsprechenden 2-[$R^2(CH_2)_m$]-2-hydroxy-, 3-[$R^2(CH_2)_m$]-3-hydroxy- oder 4-[$R^2(CH_2)_m$]-4-hydroxypiperidinen sowie, falls erwünscht, nachfolgende Dehydratisierung zu 2-, 3- oder 4-[$R^2(CH_2)_m$]-2,3- oder -3,4-dehydro-piperidinen und Hydrierung zu 2-, 3- oder 4-[$R^2(CH_2)_m$]-piperidinen. Verbindungen der Formel III ($X^2$ und $x^3$ = jeweils X) sind z.B. herstellbar durch Reduktion entsprechender Diester zu Diolen der Formel HO-R-OH (III, $X^2$ = $X^3$ = OH) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ox-$C_nH_{2n}$-$NH_2$ bzw. der Verbindung der Formel IIIa kann günstig sein. Die Reaktionstemperatur liegt je nach den angewendeten Bedingungen zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu der katalytischen Hydrierung, des nascierenden Wasserstoffs oder bestimmter komplexer Metallhydride wie $NaBH_4$.

Eine Gruppe bevorzugter Ausgangsstoffe für die Reduktion entspricht der Formel VI

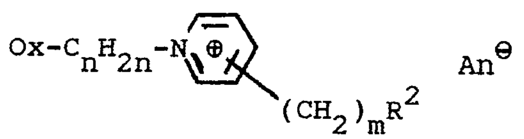 VI

worin
Ox, $R^2$, m und n die angegebenen Bedeutungen haben und $An^{\ominus}$ ein Anion einer starken Säure, bevorzugt $Cl^{\ominus}$ oder $Br^{\ominus}$, bedeutet. Verbindungen der Formel VI sind z.B. herstellbar durch Umsetzung einer Verbindung der Formel II mit einem 2-, 3- oder 4-[$R^2(CH_2)_m$]-pyridin unter den Bedingungen, die oben für die Umsetzung von II und III angegeben sind.

Für die katalytische Hydrierung sind als Katalysatoren beispielsweise Edelmetall-, Nickel- und Kobaltkatalysatoren geeignet. Die Edelmetallkatalysatoren können auf Trägern (z.B. Platin oder Palladium auf Kohle, Palladium auf Calciumcarbonat oder Strontiumcarbonat), als Oxidkatalysatoren (z.B. Platinoxid), oder als feinteilige Metallkatalysatoren vorliegen. Nickel- und Kobaltkatalysatoren werden zweckmäßig als Raney-Metalle, Nickel auch auf Kieselgur oder Bimsstein als Träger eingesetzt. Die Hydrierung kann bei Raumtemperatur und Normaldruck oder auch bei erhöhter Temperatur und/oder erhöhtem Druck durchgeführt werden. Vorzugsweise arbeitet man bei Drucken zwischen 1 und 100 at und bei Temperaturen zwischen -80 und +150°, in erster Line zwischen Raumtemperatur und +100°. Die Umsetzung wird zweckmäßig im sauren, neutralen oder basischen Bereich und in Gegenwart eines Lösungsmittels, wie Wasser, Methanol, Ethanol, Isopropanol, n-Butanol, Ethylacetat, Dioxan, Essigsäure oder THF durchgeführt; man kann auch Gemische dieser Lösungsmittel untereinander anwenden.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalischwäßriger Lösung, gegebenenfalls unter Zusatz von Ethanol verwenden. Auch Natrium- oder Aluminiumamalgam in wäßrigalkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner komplexe Metallhydride, wi $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Eine katalytische Hydrierung von Verbindungen der Formel VI liefert in der Regel die entsprechenden Piperidinderivate. Reduziert man dagegen die Verbindungen der Formel VI mit $NaBH_4$, so entstehen hauptsächlich die entsprechenden 1,2,3,6-Tetrahydropyridinderivate.

Man gelangt ferner zu Verbindungen der Formel I, worin NR eine 3- oder 4-[$R^2(CH_2)_m$]-1,2,3,6-tetrahydropyridylgruppe ist, indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1$ = X) mit einer Verbindung der Formel $HR^5$, worin $R^5$ die angegebene Bedeutung hat.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin;· als Lösungsmittel benutzt man z.B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel Vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z.B. die der

Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Verbindungen der Formel I sind ferner durch Umsetzung von Aminoalkoholen der Formel V mit reaktionsfähigen Derivaten der Kohlensäure erhältlich. Als solche eignen sich bevorzugt Dialkylcarbonate wie Dimethyl- oder Diethylcarbonat, Chlorameisensäureester wie Chlorameisensäure-methyl- oder -ethylester, N,N'-Carbonyldiimidazol oder Phosgen. Die Umsetzung gelingt zweckmäßig in Gegenwart eines inerten Lösungsmittels, vorzugsweise eine halogenierten Kohlenwasserstoffs wie Chloroform, eines Kohlenwasserstoffs wie Toluol oder eines Amids wie DMF bei Temperaturen zwischen etwa 20 und etwa 200, vorzugsweise zwischen 100 und 150°. Das Kohlensäurederivat wird zweckmäßig im Überschuß eingesetzt.

Weiterhin kann man gegebenenfalls eine Verbindung der Formel I nach an sich bekannten Methoden in eine andere Verbindung der Formel I umwandeln.

So können Ether (O-Alkylderivate) gespalten werden, wobei die entsprechenden Hydroxyderivate entstehen. Z.B. kann man die Ether spalten durch Behandeln mit Dimethylsulfid-Bortribromid-Komplex, z.B. in Toluol, 1,2-Dichlorethan, THF oder Dimethylsulfoxid, durch Verschmelzen mit Pyridin- oder Anilinhydrohalogeniden, vorzugsweise Pyridinhydrochlorid, bei etwa 150-250°, mit HBr/Essigsäure oder mit Al-trihalogeniden in chlorierten Kohlenwasserstoffen wie 1,2-Dichlorethan.

Die Verbindungen der Formel I können ein oder mehrere Asymmetriezentren besitzen. Sie können daher bei ihrer Herstellung als Racemate oder falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäuren, Salpetersäure, Sulfaminsäure, ferner organische Säuren, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Säureadditionssalze, die nicht physiologisch unbedenklich sind (z.B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Heirbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere

Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Schizophrenie und psychoreaktiven Störungen und Psychopathien, von Depressionen, von schweren chronischen Schmerzen und von Krankheiten, die mit erhöhtem Blutdruck einhergehen. Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

Beispiel 1

Man kocht 4,10 g 5-(2-Methansulfonyloxyethyl)-3-p-methoxyphenyl-oxazolidin-2-on [F. 61-64°; erhältlich durch Reaktion von 3, 4-Epoxy-1-butanol mit N-Benzyl-p-methoxyanilin zu 1-(N-Benzyl-p-methoxy-anilino)-butan-2,4-diol (Harz), Hydrogenolyse zu 1-p-Methoxyanilinobutan-2,4-diol (Harz), Umsetzung mit Diethylcarbonat zu 5-(2-Hydroxyethyl)-3-p-methoxyphenyl-oxazolidin-2-on (F. 77-78°) und Reaktion mit $CH_3SO_2Cl$] mit 3,03 g 4-p-Chlorphenyl-4-hydroxy-piperidin, 2,16 g Kaliumjodid und 1,8 g Kaliumcarbonat in 100 ml Acetonitril 16 Std., kühlt ab, arbeitet wie üblich auf und erhält 3-p-Methoxyphenyl-5-[2-(4-p-chlorphenyl-4-hydroxy-piperidino-)-ethyl]-oxazolidin-2-on, F. 95-97°.

Analog erhält man aus den entsprechenden 5-(2-Hydroxy-ethyl)-3-$R^1$-oxazolidin-2-onen, z.B.:
5-(2-Hydroxyethyl)-3-p-fluorphenyl-oxazolidin-2-on
5-(2-Hydroxyethyl)-3-p-chlorphenyl-oxazolidin-2-on
5-(2-Hydroxyethyl)-3-m-trifluormethylphenyl-oxazolidin-2-on
5-(2-Hydroxyethyl)-3-(3,4-dimethoxyphenyl)-oxazolidin-2-on
über die entsprechenden 5-(2-Methansulfonyloxyethyl)-,
5-(2-Chlorethyl)- oder 5-(2-Bromethyl)-3-$R^1$-oxazolidin-2-one der Formel II, z.B.:
5-(2-Chlorethyl)-3-p-methoxyphenyl-oxazolidin-2-on
5-(2-Methansulfonyloxyethyl)-3-p-fluorphenyl-oxazolidin-2-on
5-(2-Methansulfonyloxyethyl)-3-p-chlorphenyl-oxazolidin-2-on
5-(2-Methansulfonyloxyethyl)-3-m-trifluormethylphenyl-oxazolidin-2-on
5-(2-Methansulfonyloxyethyl)-3-(3,4-dimethoxyphenyl)-oxazolidin-2-on
mit den entsprechenden Piperidinderivaten der Formel III (worin $X^2$ und $X^3$ zusammen NH bedeuten):
3-Phenyl-5-[2-(4-phenyl-piperidino)-ethyl]-oxazolidin-2-on
3-Phenyl-5-[2-(2-benzyl-piperidino)-ethyl]-oxazolidin-2-on
3-m-Tolyl-5-[2-(2-benzyl-piperidino)-ethyl]-oxazolidin-2-on
3-o-Methoxyphenyl-5-[2-(2-benzyl-piperidino)-ethyl]-oxazolidin-2-on
3-m-Methoxyphenyl-5-[2-(2-benzyl-piperidino)-ethyl]-oxazolidin-2-on
3-p-Methoxyphenyl-5-[2-(2-benzyl-piperidino)-ethyl]-oxazolidin-2-on
3-p-Fluorphenyl-5-[2-(2-benzyl-piperidino)-ethyl]-oxazolidin-2-on
3-p-Chlorphenyl-5-[2-(2-benzyl-piperidino)-ethyl]-oxazolidin-2-on

3-m-Trifluormethylphenyl-5-[2-(2-benzyl-piperidino)-ethyl]-oxazolidin-2-on
3-(3,4-Dimethoxyphenyl)-5-[2-(2-benzyl-piperidino)-ethyl]-oxazolidin-2-on
3-Phenyl-5-[2-(3-benzyl-piperidino)-ethyl]-oxazolidin-2-on
3-m-Tolyl-5-[2-(3-benzyl-piperidino)-ethyl]-oxazolidin-2-on
3-o-Methoxyphenyl-5-[2-(3-benzyl-piperidino)-ethyl]-oxazolidin-2-on
3-m-Methoxyphenyl-5-[2-(3-benzyl-piperidino)-ethyl]-oxazolidin-2-on
3-p-Methoxyphenyl-5-[2-(3-benzyl-piperidino)-ethyl]-oxazolidin-2-on
3-p-Fluorphenyl-5-[2-(3-benzyl-piperidino)-ethyl]-oxazolidin-2-on
3-p-Chlorphenyl-5-[2-(3-benzyl-piperidino)-ethyl]-oxazolidin-2-on
3-m-Trifluormethylphenyl-5-[2-(3-benzyl-piperidino)-ethyl]-oxazolidin-2-on
3-(3,4-Dimethoxyphenyl)-5-[2-(3-benzyl-piperidino)-ethyl]-oxazolidin-2-on
3-Phenyl-5-[2-(4-benzyl-piperidino)-ethyl]-oxazolidin-2-on
3-m-Tolyl-5-[2-(4-benzyl-piperidino)-ethyl]-oxazolidin-2-on
3-o-Methoxyphenyl-5-[2-(4-benzyl-piperidino)-ethyl]-oxazolidin-2-on
3-m-Methoxyphenyl-5-[2-(4-benzyl-piperidino)-ethyl]-oxazolidin-2-on
3-p-Methoxyphenyl-5-[2-(4-benzyl-piperidino)-ethyl]-oxazolidin-2-on
3-p-Fluorphenyl-5-[2-(4-benzyl-piperidino)-ethyl]-oxazolidin-2-on
3-p-Chlorphenyl-5-[2-(4-benzyl-piperidino)-ethyl]-oxazolidin-2-on
3-m-Trifluormethylphenyl-5-[2-(4-benzyl-piperidino)-ethyl]-oxazolidin-2-on
3-(3,4-Dimethoxyphenyl)-5-[2-(4-benzyl-piperidino)-ethyl]-oxazolidin-2-on
3-Phenyl-5-[2-(4-hydroxy-4-phenyl-piperidino)-ethyl]-oxazolidin-2-on
3-m-Tolyl-5-[2-(4-hydroxy-4-phenyl-piperidino)-ethyl]-oxazolidin-2-on
3-o-Methoxyphenyl-5-[2-(4-hydroxy-4-phenyl-piperidino)-ethyl]-oxazolidin-2-on
3-m-Methoxyphenyl-5-[2-(4-hydroxy-4-phenyl-piperidino)-ethyl]-oxazolidin-2-on
3-p-Methoxyphenyl-5-[2-(4-hydroxy-4-phenyl-piperidino)-ethyl]-oxazolidin-2-on, F. 145-147°
3-p-Fluorphenyl-5-[2-(4-hydroxy-4-phenyl-piperidino)-ethyl]-oxazolidin-2-on
3-p-Chlorphenyl-5-[2-(4-hydroxy-4-phenyl-piperidino)-ethyl]-oxazolidin-2-on
3-m-Trifluormethylphenyl-5-[2-(4-hydroxy-4-phenyl-piperidino)-ethyl]-oxazolidin-2-on
3-(3,4-Dimethoxyphenyl)-5-[2-(4-hydroxy-4-phenyl-piperidino)-ethyl]-oxazolidin-2-on
3-Phenyl-5-[2-(4-hydroxy-4-p-methoxyphenyl-piperidino)-ethyl]-oxazolidin-2-on
3-m-Tolyl-5-[2-(4-hydroxy-4-p-methoxyphenyl-piperidino)-ethyl]-oxazolidin-2-on
3-o-Methoxyphenyl-5-[2-(4-hydroxy-4-p-methoxyphenyl-piperidino)-ethyl]-oxazolidin-2-on
3-m-Methoxyphenyl-5-[2-(4-hydroxy-4-p-methoxyphenyl-piperidino)-ethyl]-oxazolidin-2-on
3-p-Methoxyphenyl-5-[2-(4-hydroxy-4-p-methoxyphenyl-piperidino)-ethyl]-oxazolidin-2-on
3-p-Fluorphenyl-5-[2-(4-hydroxy-4-p-methoxyphenyl-piperidino)-ethyl]-oxazolidin-2-on
3-p-Chlorphenyl-5-[2-(4-hydroxy-4-p-methoxyphenyl-piperidino)-ethyl]-oxazolidin-2-on
3-m-Trifluormethylphenyl-5-[2-(4-hydroxy-4-p-methoxyphenyl-piperidino)-ethyl]-oxazolidin-2-on
3-(3,4-Dimethoxyphenyl)-5-[2-(4-hydroxy-4-p-methoxyphenyl-piperidino)-ethyl]-oxazolidin-2-on
3-Phenyl-5-[2-(4-hydroxy-4-p-fluorphenyl-piperidino)-ethyl]-oxazolidin-2-on
3-m-Tolyl-5-[2-(4-hydroxy-4-p-fluorphenyl-piperidino)-ethyl]-oxazolidin-2-on
3-o-Methoxyphenyl-5-[2-(4-hydroxy-4-p-fluorphenyl-piperidino)-ethyl]-oxazolidin-2-on
3-m-Methoxyphenyl-5-[2-(4-hydroxy-4-p-fluorphenyl-piperidino)-ethyl]-oxazolidin-2-on
3-p-Methoxyphenyl-5-[2-(4-hydroxy-4-p-fluorphenyl-piperidino)-ethyl]-oxazolidin-2-on
3-p-Fluorphenyl-5-[2-(4-hydroxy-4-p-fluorphenyl-piperidino)-ethyl]-oxazolidin-2-on
3-p-Chlorphenyl-5-[2-(4-hydroxy-4-p-fluorphenyl-piperidino)-ethyl]-oxazolidin-2-on
3-m-Trifluormethylphenyl-5-[2-(4-hydroxy-4-p-fluorphenyl-piperidino)-ethyl]-oxazolidin-2-on
3-(3,4-Dimethoxyphenyl)-5-[2-(4-hydroxy-4-p-fluorphenyl-piperidino)-ethyl]-oxazolidin-2-on
3-Phenyl-5-[2-(4-hydroxy-4-p-chlorphenyl-piperidino)-ethyl]-oxazolidin-2-on
3-m-Tolyl-5-[2-(4-hydroxy-4-p-chlorphenyl-piperidino)-ethyl]-oxazolidin-2-on
3-o-Methoxyphenyl-5-[2-(4-hydroxy-4-p-chlorphenyl-piperidino)-ethyl]-oxazolidin-2-on
3-m-Methoxyphenyl-5-[2-(4-hydroxy-4-p-chlorphenyl-piperidino)-ethyl]-oxazolidin-2-on
3-p-Fluorphenyl-5-[2-(4-hydroxy-4-p-chlorphenyl-piperidino)-ethyl]-oxazolidin-2-on
3-p-Chlorphenyl-5-[2-(4-hydroxy-4-p-chlorphenyl-piperidino)-ethyl]-oxazolidin-2-on
3-m-Trifluormethylphenyl-5-[2-(4-hydroxy-4-p-chlorphenyl-piperidino)-ethyl]-oxazolidin-2-on
3-(3,4-Dimethoxyphenyl)-5-[2-(4-hydroxy-4-p-chlorphenyl-piperidino)-ethyl]-oxazolidin-2-on
3-Phenyl-5-[2-(4-hydroxy-4-m-trifluormethylphenyl-piperidino)-ethyl]-oxazolidin-2-on
3-m-Tolyl-5-[2-(4-hydroxy-4-m-trifluormethylphenyl-piperidino)-ethyl]-oxazolidin-2-on
3-o-Methoxyphenyl-5-[2-(4-hydroxy-4-m-trifluormethylphenyl-piperidino)-ethyl]-oxazolidin-2-on

3-m-Methoxyphenyl-5-[2-(4-hydroxy-4-m-trifluormethylphenyl-piperidino)-ethyl]-oxazolidin-2-on
3-p-Methoxyphenyl-5-[2-(4-hydroxy-4-m-trifluormethylphenyl-piperidino)-ethyl]-oxazolidin-2-on
3-p-Fluorphenyl-5-[2-(4-hydroxy-4-m-trifluormethylphenyl-piperidino)-ethyl]-oxazolidin-2-on
3-p-Chlorphenyl-5-[2-(4-hydroxy-4-m-trifluormethylphenyl-piperidino)-ethyl]-oxazolidin-2-on
3-m-Trifluormethylphenyl-5-[2-(4-hydroxy-4-m-trifluormethylphenyl-piperidino)-ethyl]-oxazolidin-2-on
3-(3,4-Dimethoxyphenyl)-5-[2-(4-hydroxy-4-m-trifluormethylphenyl-piperidino)-ethyl]-oxazolidin-2-on
3-Phenyl-5-[2-(4-benzyl-4-hydroxy-piperidino)-ethyl]-oxazolidin-2-on
3-Phenyl-5-[2-(4-hydroxy-4-(2-thienyl)-piperidino)-ethyl]-oxazolidin-2-on, F. 132-134°
3-Phenyl-5-[2-(4-benzoyl-piperidino)-ethyl]-oxazolidin-2-on
3-m-Tolyl-5-[2-(4-benzoyl-piperidino)-ethyl]-oxazolidin-2-on
3-o-Methoxyphenyl-5-[2-(4-benzoyl-piperidino)-ethyl]-oxazolidin-2-on
3-m-Methoxyphenyl-5-[2-(4-benzoyl-piperidino)-ethyl]-oxazolidin-2-on
3-p-Methoxyphenyl-5-[2-(4-benzoyl-piperidino)-ethyl]-oxazolidin-2-on
3-p-Fluorphenyl-5-[2-(4-benzoyl-piperidino)-ethyl]-oxazolidin-2-on
3-p-Chlorphenyl-5-[2-(4-benzoyl-piperidino)-ethyl]-oxazolidin-2-on
3-m-Trifluormethylphenyl-5-[2-(4-benzoyl-piperidino)-ethyl]-oxazolidin-2-on
3-(3,4-Dimethoxyphenyl)-5-[2-(4-benzoyl-piperidino)-ethyl]-oxazolidin-2-on
3-Phenyl-5-[2-(4-(2-oxobenzimidazolin-1-yl)-piperidino)-ethyl]-oxazolidin-2-on
3-m-Tolyl-5-[2-(4-(2-oxobenzimidazolin-1-yl)-piperidino)-ethyl]-oxazolidin-2-on
3-o-Methoxyphenyl-5-[2-(4-(2-oxobenzimidazolin-1-yl)-piperidino)-ethyl]oxazolidin-2-on
3-m-Methoxyphenyl-5-[2-(4-(2-oxobenzimidazolin-1-yl)-piperidino)-ethyl]-oxazolidin-2-on
3-p-Methoxyphenyl-5-[2-(4-(2-oxobenzimidazolin-1-yl)-piperidino)-ethyl]-oxazolidin-2-on, 184-186°
3-p-Fluorphenyl-5-[2-(4-(2-oxobenzimidazolin-1-yl)-piperidino)-ethyl]-oxazolidin-2-on
3-p-Chlorphenyl-5-[2-(4-(2-oxobenzimidazolin-1-yl)-piperidino)-ethyl]-oxazolidin-2-on
3-m-Trifluormethylphenyl-5-[2-(4-(2-oxobenzimidazolin-1-yl)-piperidino)-ethyl]-oxazolidin-2-on
3-(3,4-Dimethoxyphenyl)-5-[2-(4-(2-oxobenzimidazolin-1-yl)-piperidino)-ethyl]-oxazolidin-2-on
4-Phenyl-5-[2-(4-(2-thioxobenzimidazolin-1-yl)-piperidino)-ethyl]-oxazolidin-2-on
3-m-Tolyl-5-[2-(4-(2-thioxobenzimidazolin-1-yl)-piperidino)-ethyl)-oxazolidin-2-on
3-o-Methoxyphenyl-5-[2-(4-(2-thioxobenzimidazolin-1-yl)-piperidino)-ethyl]-oxazolidin-2-on
3-m-Methoxyphenyl-5-[2-(4-(2-thioxobenzimidazolin-1-yl)-piperidino)-ethyl]-oxazolidin-2-on
3-p-Methoxyphenyl-5-[2-(4-(2-thioxobenzimidazolin-1-yl)-piperidino)-ethyl]-oxazolidin-2-on
3-p-Fluorphenyl-5-[2-(4-(2-thioxobenzimidazolin-1-yl)-piperidino)-ethyl]-oxazolidin-2-on
3-p-Chlorphenyl-5-[2-(4-(2-thioxobenzimidazolin-1-yl)-piperidino)-ethyl]-oxazolidin-2-on
3-m-Trifluormethylphenyl-5-[2-(4-(2-thioxobenzimidazolin-1-yl)-piperidino)-ethyl]-oxazolidin-2-on
3-(3,4-Dimethoxyphenyl)-5-[2-(4-(2-thioxobenzimidazolin-1-yl)-piperidino)-ethyl]-oxazolidin-2-on
1-Phenyl-4-oxo-8-[2-(3-phenyl-oxazolidin-2-on-5-yl)-ethyl]-1,3,8-triazaspiro[4,5]decan
1-Phenyl-4-oxo-8-[2-(3-m-tolyl-oxazolidin-2-on-5-yl)-ethyl]-1,3,8-triazaspiro[4,5]decan
1-Phenyl-4-oxo-8-[2-(3-o-methoxyphenyl-oxazolidin-2-on-5-yl)-ethyl]-1,3,8-triazaspiro[4,5]decan
1-Phenyl-4-oxo-8-[2-(3-m-methoxyphenyl-oxazolidin-2-on-5-yl)-ethyl]-1,3,8-triazaspiro[4,5]decan
1-Phenyl-4-oxo-8-[2-(3-p-methoxyphenyl-oxazolidin-2-on-5-yl)-ethyl]-1,3,8-triazaspiro[4,5]decan
1-Phenyl-4-oxo-8-[2-(3-p-fluorphenyl-oxazolidin-2-on-5-yl)-ethyl]-1,3,8-triazaspiro[4,5]decan
1-Phenyl-4-oxo-8-[2-(3-p-chlorphenyl-oxazolidin-2-on-5-yl)-ethyl]-1,3,8-triazaspiro[4,5]decan
1-Phenyl-4-oxo-8-[2-(3-m-trifluormethylphenyl-oxazolidin-2-on-5-yl)-ethyl]-1,3,8-triazaspiro[4,5]decan
1-Phenyl-4-oxo-8-[2-(3-(3,4-dimethoxyphenyl)-oxazolidin-2-on-5-yl)-ethyl]-1,3,8-triazaspiro[4,5]decan.

Beispiel 2

Analog Beispiel 1 erhält man mit 5-(3-Methansulfonyloxy-propyl)-3-p-methoxyphenyl-oxazolidin-2-on [F. 75-76°; erhältlich aus 4-Penten-1-ol über 4-Penten-1-olbenzoat, 4,5-Epoxypentan-1-ol-benzoat, 5-p-Methoxyanilino-pentan-1,4-diol-1-benzoat, 5-(3-Benzoyloxypropyl)-3-p-methoxy-phenyl-oxazolidin-2-on und 5-(3-Hydroxypropyl)-3-p-methoxyphenyl-oxazolidin-2-on (F. 78-79°)]:
3-p-Methoxyphenyl-5-[3-(4-p-chlorphenyl-4-hydroxy-piperidino)-propyl]-oxazolidin-2-on, F. 142-145°
3-p-Methoxyphenyl-5-[3-(4-phenyl-4-hydroxy-piperidino)-propyl]-oxazolidin-2-on
3-p-Methoxyphenyl-5-[3-(4-p-methoxyphenyl-4-hydroxy-piperidino)-propyl]-oxazolidin-2-on
3-p-Methoxyphenyl-5-[3-(4-p-fluorphenyl-4-hydroxy-piperidino)-propyl]-oxazolidin-2-on
3-p-Methoxyphenyl-5-[3-(4-m-trifluormethylphenyl-4-hydroxy-piperidino)-propyl]-oxazolidin-2-on
3-p-Methoxyphenyl-5-[3-(4-(2-thienyl)-4-hydroxy-piperidino)-4-hydroxy-piperidino)-propyl]-oxazolidin-2-on, F.

148-150°.

Beispiel 3

Analog Beispiel 1 erhält man aus (S)-5-(2-Methansulfonyloxyethyl)-3-p-methoxyphenyl-oxazolidin-2-on [erhältlich aus (S)-3,4-Epoxy-1-butanol] das (S)-3-p-Methoxyphenyl-5-[2-(4-p-chlorphenyl-4-hydroxy-piperidino)-ethyl]-oxazolidin-2-on.

Beispiel 4

Ein Gemisch von 2,06 g 5-(2-Aminoethyl)-3-phenyl-oxazolidin-2-on [erhältlich durch Reaktion von 5-(2-chlorethyl)-3-phenyl-oxazolidin-2-on mit Phthalimidkalium und nachfolgende Hydrolyse] und 2,15 g 1,5-Dichlor-3-phenyl-2-penten in 40 ml Aceton und 40 ml Wasser wird 24 Stunden gekocht und wie üblich aufgearbeitet. Man erhält 3-Phenyl-5-[2-(4-phenyl-1,2,3,6-tetrahydro-1-pyridyl)-ethyl-oxazolidin-2-on.

Beispiel 5

Zu einer Lösung von 4,55 g 1-[2-(3-p-Methoxyphenyl-oxazolidin-2-on-5-yl)-ethyl]-4-phenyl-pyridiniumbromid (erhältlich aus 3-p-Methoxyphenyl-5-(2-bromethyl)-oxazolidin-2-on und 4-Phenylpyridin) in 50 ml 1 n NaOH gibt man unter Rühren 1 g $NaBH_4$ in 20 ml Wasser und rührt danach 3 Stunden bei 60°. Nach üblicher Aufarbeitung erhält man 3-p-Methoxyphenyl-5-[2-(4-phenyl-1,2,3,6-tetrahydro-1-pyridyl)-ethyl]-oxazolidin-2-on.

Beispiel 6

Man erhitzt 1 g 3-p-Methoxyphenyl-5-[2-(4-hydroxy-4-phenyl-piperidino)-ethyl]-oxazolidin-2-on mit 10 ml 1 n Salzsäure 2 Stunden auf 100°, arbeitet wie üblich auf und erhält 3-p-Methoxyphenyl-5-[2-(4-phenyl-1,2,3,6-tetrahydro-1-pyridyl)-ethyl]-oxazolidin-2-on.

Beispiel 7

Ein Gemisch von 3,7 g 4-Hydroxy-1-(3-hydroxy-4-p-methoxyanilino-butyl)-4-phenyl-piperidin (erhältlich durch Reaktion von p-Methoxyanilin mit 3,4-Epoxybuttersäureethylester zu 3-Hydroxy-4-p-methoxyanilino-buttersäureethylester, Reduktion mit $LiAlH_4$ zu 4-p-Methoxyanilino-1,3-propandiol, Dehydratisierung zum Epoxid und Umsetzung mit 4-Hydroxy-4-phenyl-piperidin), 1,5 g Diethylcarbonat und 50 ml DMF wird 4 Stunden auf 120° erhitzt. Nach Eindampfen und üblicher Aufarbeitung erhält man 3-p-Methoxyphenyl-5-[2-(4-hydroxy-4-phenyl-piperidino)-ethyl]-oxazolidin-2-on,

Beispiel 8

Ein Gemisch von 10 g 3-p-Methoxyphenyl-5-[2-(4-phenyl-1,2,3,6-tetrahydro-1-pyridyl)-ethyl]-oxazolidin-2-on und 10 g Pyridinhydrochlorid wird 3 Stunden bei 160° gerührt. Nach üblicher Aufarbeitung erhält man 3-p-Hydroxyphenyl-5-[2-(4-phenyl-1,2,3,6-tetrahydropyridyl-1-methyl)-oxazolidin-2-on.

Beispiel 9

Man tropft eine Suspension von 3,8 g 3-p-Methoxyphenyl-5-[2-(4-benzyl-piperidino)-ethyl]-oxazolidin-2-on in 50 ml 1,2-Dichlorethan zu einer siedenden Lösung von 15,6 g Dimethylsulfid-Bortribromid-Komplex in 50 ml 1,2-Dichlorethan, kocht noch 30 Minuten, arbeitet wie üblich auf und erhält 3-p-Hydroxyphenyl-5-[2-(4-benzyl-piperidino)-ethyl]-oxazolidin-2-on.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 3-p-Methoxyphenyl-5-[2-(4-hydroxy-4-p-chlorphenyl-piperidino)-ethyl]-oxazolidin-2-on, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 3-p-Methoxyphenyl-5-[2-(4-hydroxy-4-phenyl-piperidino)-ethyl]-oxazolidin-2-on werden in üblicher Weise in Hartgelatinekapseln gefüllt, so daß jede Kapsel 20 mg des Wirkstoffs enthält.

Beispiel D: Ampullen

Eine Lösung von 1 kg 3-p-Methoxyphenyl-5-[2-(4-benzyl-piperidino)-ethyl]-oxazolidin-2-on-hydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Oxazolidinone der Formel I

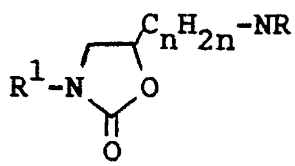

I

worin

R

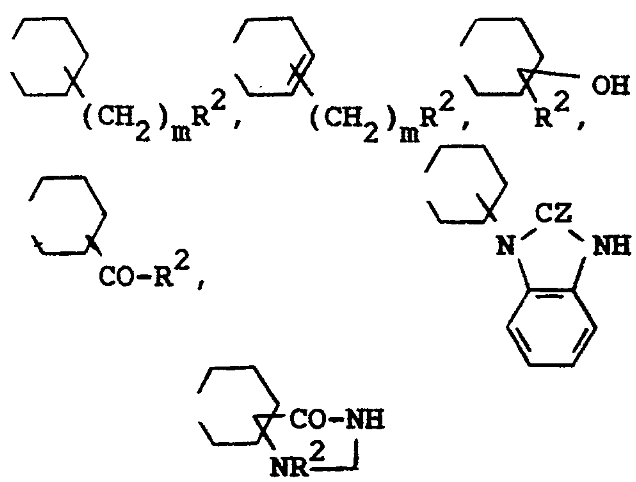

oder

Z O oder S,

$R^1$ und $R^2$ jeweils unsubstituierte oder ein- oder zweifach durch Alkyl, Alkoxy, Alkylthio, Alkylsulfinyl oder Alkylsulfonyl mit jeweils 1-4 C-Atomen, Alkanoyloxy und/oder Alkanoylamino mit jeweils 1-6 C-Atomen, F, Cl, Br, OH und/oder $CF_3$ substituierte Phenyl- oder ein- oder zweikernige, 1-4 Heteroatome enthaltende Heteroarylreste,

m 0 oder 1 und

n 2 oder 3

bedeuten,

sowie deren Salze.

**2.**

a) 3-p-Methoxyphenyl-5-[2-(4-hydroxy-4-phenylpiperidino)-ethyl]-oxazolidin-2-on;

b) 3-p-Methoxyphenyl-5-[2-(4-hydroxy-4-p-methoxyphenylpiperidino)-ethyl]-oxazolidin-2-on;

c) 3-p-Methoxyphenyl-5-[2-(4-hydroxy-4-p-chlorphenyl-piperidino)-ethyl]-oxazolidin-2-on;

d) 3-p-Methoxyphenyl-5-[2-(4-hydroxy-4-(2-thienyl)-piperidino)-ethyl]-oxazolidin-2-on.

**3.** Verfahren zur Herstellung von Oxazolidinonen der Formel I sowie von deren Salzen, dadurch gekennzeichnet, daß man eine Verbindung der Formel II

$Ox\text{-}C_nH_{2n}\text{-}X^1$ II

worin

Ox

den Rest

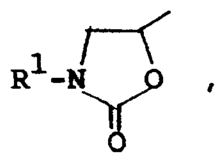,

$X^1$ X oder $NH_2$,

X Cl, Br, J, OH oder eine reaktionsfähig funktionell abgewandelte OH-Gruppe bedeuten und

$R^1$ und n die angegebenen Bedeutungen haben,

mit einer Verbindung der Formel III

$X^2\text{-}R\text{-}X^3$ III

worin

$X^2$ und $X^3$ gleich oder verschieden sind und, falls $X^1 = NH_2$ ist, jeweils X, andernfalls zusammen NH bedeuten und

R die angegebene Bedeutung hat,

umsetzt

und/oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

und/oder daß man zur Herstellung einer Verbindung der Formel I, worin

R 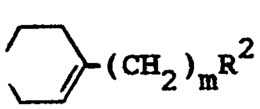 oder 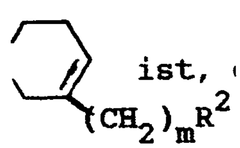 ist,

eine

Verbindung der Formel IV

$OxC_nH_{2n}\text{-}R^5$

worin

$R^5$

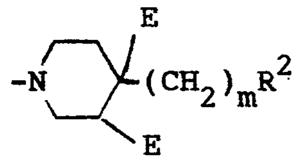

oder

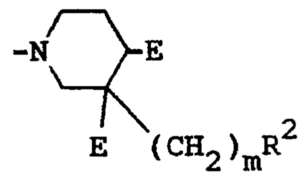

der eine Rest E X, CN oder $NH_2$,
der andere Rest E H bedeutet und
Ox, $R^2$, X und n die angegebenen Bedeutungen haben
mit einem HE-abspaltenden Mittel behandelt
oder daß man eine Verbindung der Formel V

$R^1$-NH-$CH_2$-CHOH-$C_nH_{2n}$-NR V

worin R, $R^1$ und n die angegebenen Bedeutungen haben, mit einem reaktionsfähigen Derivat der Kohlensäure umsetzt
und/oder daß man gegebenenfalls in einer Verbindung der Formel I eine O-Alkylgruppe unter Bildung einer OH-Gruppe spaltet und/oder eine Verbindung der Formel I durch Reduktion in eine andere Verbindung der Formel I umwandelt
und/oder daß man eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und/oder ihre physiologisch unbedenklichen Salze zur Bekämpfung von Krankheiten.

7. Verwendung von Verbindungen der Formel I und/oder ihren physiologisch unbedenklichen Salzen bei der Bekämpfung von Krankheiten.

8. Verwendung von Verbindungen der Formel I und/oder ihren physiologisch unbedenklichen Salzen zur Herstellung von Arzneimitteln.